# EUROPEAN PATENT SPECIFICATION

(11) **EP 3 856 357 B1**
(45) Date of publication and mention of the grant of the patent: **10.09.2025**
(21) Application number: 18804867.2
(22) Date of filing: 02.11.2018
(51) Int. Cl.: A61Q 11/00, A61K 8/22, A61K 8/23, A61K 8/31, A61K 8/24, A61K 8/81, A61K 8/891, A61K 8/895

(54) **WHITENING ORAL CARE COMPOSITION BASED ON A CROSS-LINKED PVP-H2O2 COMPLEX, A PEROXYSULFATE, A HYDROPHOBIC BASE AND SODIUM TRIPOLYPHOSPHATE**
BLEICHENDE MUNDPFLEGEZUSAMMENSETZUNG BASIEREND AUF EINEM VERNETZTEN PVP-H2O2-KOMPLEX, EINEM PEROXYSULFAT, EINER HYDROPHOBEN BASIS UND NATRIUMTRIPOLYPHOSPHAT
COMPOSITION DE SOIN BUCCAL POUR BLANCHIMENT À BASE D'UN COMPLEXE PVP-H2O2 RÉTICULÉ, D'UN PEROXYSULFATE, D'UNE BASE HYDROPHOBE ET DE TRIPOLYPHOSPHATE DE SODIUM

(43) Date of publication of application: 04.08.2021
(60) Divisional application: 25193360.2
(73) Proprietor: Colgate-Palmolive Company, New York, NY 10022 (US)
(72) Inventor: GALIYARA, Tanaz, Brunswick, New Jersey 08816 (US); CHOPRA, Suman, Monroe, New Jersey 08831 (US)
(74) Representative: Wibbelmann, Jobst
(86) International application number: PCT/US2018/058968
(87) International publication number: WO 2020/091808

(56) References cited:
- WO-A1-2019/036696
- US-A1- 2017 172 864
- US-A1- 2017 172 866

## Description

### BACKGROUND

Oral care products with teeth whitening attributes use a variety of active ingredients to remove stains or whiten teeth. The most commonly used whitening active ingredients are peroxide sources, such as hydrogen peroxide. These products typically contain substantial amounts of the peroxide source, usually up to about 10% hydrogen peroxide. However, in high concentrations, hydrogen peroxide can be irritating to the teeth and gums and, in addition, hydrogen peroxide is an unstable molecule that is prone to decomposition, especially in aqueous environments.

Accordingly, there is a desire for oral care composition using lower concentrations of a peroxide source, and/or alternative oxidizing agents, that are stable, provide effective teeth whitening, and have good microrobustness. US 2017/0172864 A1 discloses tooth whitening compositions comprising a peroxide complex comprising hydrogen peroxide and an N-vinyl heterocyclic polymer; sodium percarbonate; and at least one hydrophobic polymer carrier, wherein the hydrogen peroxide of the peroxide complex is present in the tooth whitening composition in an amount ranging from about 3% to about 5% by weight. US 2017/0172866 A1 discloses oral care whitening compositions comprising a non-aqueous phase comprising a peroxysulfate whitening agent, a hydrophobic base and at least one hydrophilic polymer, and optionally an aqueous phase comprising at least one hydrophilic polymer.

### BRIEF SUMMARY

This summary is intended merely to introduce a simplified summary of some aspects of one or more implementations of the present disclosure. Further areas of applicability of the present invention will become apparent from the detailed description provided hereinafter. This summary is not an extensive overview, nor is it intended to identify key or critical elements of the present teachings, nor to delineate the scope of the disclosure. Rather, its purpose is merely to present one or more concepts in simplified form as a prelude to the detailed description below.

The foregoing and/or other aspects and utilities embodied in the present disclosure may be achieved by providing an oral care composition, including a peroxide source including a cross-linked polyvinylpyrrolidone-hydrogen peroxide complex (cPVP-H₂O₂ complex) in an amount configured to provide from 0.01 weight % to 6.0 weight % hydrogen peroxide based on a total weight of the oral care composition; a peroxysulfate whitening agent; from 20 weight % to 80 weight % of a hydrophobic base based on the total weight of the oral care composition; and from 0.05 weight % to 3 weight % sodium tripolyphosphate (STPP).

The peroxysulfate whitening agent may include potassium peroxymonosulfate (MPS), and an amount of the peroxysulfate whitening agent may be configured to provide from about 0.01 weight % to about 4 weight % MPS based on the total weight of the oral care composition.

The hydrophobic base may include one or more of a hydrophobic polymer, a pressure sensitive adhesive, and a hydrocarbon.

The hydrophobic polymer may include polydimethylsiloxane.

The pressure sensitive adhesive may be a silicon copolymer prepared by condensing a silicone resin with a polydiorganosiloxane.

The hydrocarbon may be petrolatum.

The hydrophobic base may include from about 30 weight % to about 70 weight % hydrophobic polymer based on the total weight of the oral care composition; from about 20 weight % to about 40 weight % pressure sensitive adhesive based on the total weight of the oral care composition; and from about 10 weight % to about 30 weight % hydrocarbon based on the total weight of the oral care composition.

The oral care composition may further include about 10 weight % to about 30 weight % of a dental surface adhesion agent.

The dental surface adhesion agent may include a polyvinylpyrrolidone (PVP) polymer, including either linear PVP or cross-linked PVP (cPVP), and mixture thereof.

The dental surface adhesion agent may be one or more of anionic polymers, cationic polymers, and neutral polar polymers, such as polyacrylate polymers, copolymers of methyl vinyl ether with maleic acid or anhydride, and linear PVP or cross-linked PVP, and any mixtures thereof.

The oral care composition may be substantially anhydrous.

The oral care composition may have less than 5 weight % water based on the total weight of the oral care composition.

The oral care composition may not comprise a surfactant.

The oral care composition may not comprise a polyethylene thickener.

The oral care composition may not comprise a source of fluoride ions.

A viscosity of the oral care composition may be from about 1,000 centipoise (cPs) to about 900,000 cPs.

The oral care composition may not comprise cetylpyridinium chloride.

The oral care composition may not comprise any antimicrobial or preservative agents other than STPP.

The oral care composition may be embodied as a gel configured to be maintained on a surface of a tooth for from about 10 minutes to about 120 minutes.

The oral care composition may have a Hershel-Bulkley rate index of less than 0.7.

The foregoing and/or other aspects and utilities embodied in the present disclosure may also be achieved by providing a method of whitening a tooth including applying the oral care composition described above to a tooth of a mammal.

The oral care composition may be sufficiently viscous to form an adherent, continuous layer on a dental surface and deliver an effective amount the peroxide source and the peroxysulfate whitening agent to a tooth surface.

### DETAILED DESCRIPTION

Reference will now be made in detail to the various implementations in the present disclosure, examples of which may be illustrated in any accompanying drawings and figures. The various implementations are described below to provide a more complete understanding of the components, processes, compositions, and apparatuses disclosed herein. Any examples given are intended to be illustrative, and not restrictive. However, it will be apparent to one of ordinary skill in the art that the invention may be practiced without these specific details. In other instances, well-known methods, procedures, and components have not been described in detail so as not to unnecessarily obscure aspects of the various implementations.

Throughout the specification and claims, the following terms take the meanings explicitly associated herein, unless the context clearly dictates otherwise. Phrases, such as "in an implementation," "in certain implementations," and "in some implementations," as used herein do not necessarily refer to the same implementation(s), though they may. Furthermore, the phrases "in another implementation" and "in some other implementations" as used herein do not necessarily refer to a different implementation, although they may.

As used herein, the term "or" is an inclusive operator, and is equivalent to the term "and/or," unless the context clearly dictates otherwise. The term "based on" is not exclusive and allows for being based on additional factors not described, unless the context clearly dictates otherwise. In the specification, the recitation of "at least one of A, B, and C," includes implementations containing A, B, or C, multiple examples of A, B, or C, or combinations of A/B, A/C, B/C, A/B/B/ B/B/C, A/B/C, etc. In addition, throughout the specification, the meaning of "a," "an," and "the" include plural references. The meaning of "in" includes "in" and "on."

It will also be understood that, although the terms first, second, etc. may be used herein to describe various elements, these elements should not be limited by these terms. These terms are only used to distinguish one element from another. For example, a first object, component, or step could be termed a second object, component, or step, and, similarly, a second object, component, or step could be termed a first object, component, or step, without departing from the scope of the invention. The first object, component, or step, and the second object, component, or step, are both, objects, component, or steps, respectively, but they are not to be considered the same object, component, or step. It will be further understood that the terms "includes," "including," "comprises" and/or "comprising," when used in this specification, specify the presence of stated features, steps, operations, elements, and/or components, but do not preclude the presence or addition of one or more other features, steps, operations, elements, components, and/or groups thereof. Further, as used herein, the term "if" may be construed to mean "when" or "upon" or "in response to determining" or "in response to detecting," depending on the context.

All physical properties that are defined hereinafter are measured at 20° to 25° Celsius unless otherwise specified.

When referring to any numerical range of values herein, such ranges are understood to include each and every number and/or fraction between the stated range minimum and maximum, as well as the endpoints. For example, a range of 0.5% to 6% would expressly include all intermediate values of, for example, 0.6%, 0.7%, and 0.9%, all the way up to and including 5.95%, 5.97%, and 5.99%, among many others. The same applies to each other numerical property and/or elemental range set forth herein, unless the context clearly dictates otherwise.

Additionally, all numerical values are "about" or "approximately" the indicated value, and take into account experimental error and variations that would be expected by a person having ordinary skill in the art. It should be appreciated that all numerical values and ranges disclosed herein are approximate values and ranges, whether "about" is used in conjunction therewith.

Unless otherwise specified, all percentages and amounts expressed herein and elsewhere in the specification should be understood to refer to percentages by weight. The amounts given are based on the active weight of the material.

With regard to procedures, methods, techniques, and workflows that are in accordance with some implementations, some operations in the procedures, methods, techniques, and workflows disclosed herein may be combined and/or the order of some operations may be changed.

The present inventors have surprisingly discovered a stable oral care composition that uses peroxysulfuric acid or peroxysulfates as a teeth whitening agent in addition to a peroxide source. The oral care composition may also have an improved teeth whitening efficacy and may demonstrate good microrobustness.

In one implementation, the oral care composition includes one or more teeth whitening agents, an anhydrous base, and an adhesion enhancing agent. In some implementations, the oral care composition is substantially anhydrous. For example, the oral care composition may be free or substantially free of water. As used herein, "free" or "substantially free" may refer to a composition that contains less than 10.0 weight %, less than 5.0 weight %, less than 3.0 weight %, less than 1.0 weight %, less than 0.1 weight %, less than 0.05 weight %, less than 0.01 weight %, less than 0.005 weight %, or less than 0.0001 weight % water based on a total weight of the oral care composition. In other implementations, the oral care composition is hydrophobic.

In other implementations, the oral care composition may include additional ingredients common to oral care compositions, such as thickeners, flavoring agents, tartar control agents, anti-calculus agents, sweeteners, humectants, colorants, dyes, and pigments. However, in some implementations, the oral care composition does not include one or more of surfactants, humectants, abrasives, glycerin, sorbitol, fluoride ion sources, water, and dyes.

All ingredients used in the oral care compositions described herein should be orally acceptable. "Orally acceptable" means an ingredient which is present in the oral care composition as described in an amount and form which does not render the oral care composition unsafe, unpalatable, or otherwise unsuitable for use in the oral cavity.

In some implementations, the oral care composition is a viscous liquid, preferably a gel, configured to maintain its consistency during storage and enabling the oral care composition to be painted on a tooth surface with a soft applicator pen or brush. For example, the oral care composition may have a viscosity high enough to remain on a tooth surface once applied for a time sufficient to effect whitening.

In one implementation, the oral care composition is a portable viscous liquid or gel tooth whitener that can be applied to the teeth as a coated layer conveniently painted onto the tooth enamel surface. Upon application to the teeth, the applied whitening oral care composition forms an adherent layer configured to release and/or activate the teeth whitening agents over an extended period of time, e.g., from about 5 minutes to about 12 hours, to effectuate the teeth whitening.

In one implementations, the oral care composition is sufficiently viscous to form an adherent, continuous layer on a dental surface and deliver an effective amount the peroxide source and the peroxysulfate whitening agent to a tooth surface.

The oral care composition has a viscosity greater than about 1,000 centipoise (cPs) and less than about 900,000 cPs. For example, the oral care composition may have a viscosity from about 10,000 cPs to about 100,000 cPs, from about 50,000 cPs to about 900,000 cPs, from about 100,000 cPs to about 150,000 cPs, or from about 200,000 cPs to about 600,000 cPs. In one implementation, the oral care composition has a viscosity of about 120,000 cPs.

In order to ensure the ability of the oral care composition to remain on a tooth surface for a sufficient time, the oral care composition specifically does not include certain ingredients common to other oral care products. For example, the oral care composition does not include surfactants, such as orally acceptable anionic, nonionic, cationic, or amphoteric surfactants.

In other implementations, the oral care composition does not include a source of fluoride ions, such as fluoride, monofluorophosphate (MFP), or fluorosilicate salts.

In other implementations, the oral care composition does not include dental abrasives or polishing agents, such as silica (in the form of silica gel, hydrated silica or precipitated silica), alumina, insoluble phosphates, calcium carbonate, resinous abrasives, such as urea-formaldehyde condensation products, and the like.

As described above, the oral care composition includes one or more whitening agent. As used herein, a "whitening agent" is a material which effects whitening of a tooth surface to which it is applied. For example, in some implementations, the whitening agent is an oxidizing agent. In its broadest sense, "oxidizing agent" is intended to include those compounds which can accept an electron from another molecule in the environment of the oral cavity without having a deleterious or unacceptably harmful effect on the oral cavity in normal and accepted use.

In one implementation, the oral care composition includes a peroxide source and a peroxysulfate as whitening agents.

The peroxide source may include a cross-linked polyvinylpyrrolidone-hydrogen peroxide complex (cPVP-H₂O₂ complex). The cPVP-H₂O₂ complex consists of hydrogen peroxide molecules intimately bound to the polyvinylpyrrolidone backbone. The cPVP-H₂O₂ complex is available commercially as a dry powder for incorporation into whitening and oxidizing compositions and typically consists of from about 15 weight % to about 30 weight % hydrogen peroxide, for example, about 18 weight % hydrogen peroxide. The cPVP-H₂O₂ complex is available commercially as PEROXYDONE XL 10 (Ashland Inc., Covington KY).

In one implementation, the amount of cPVP-H₂O₂ complex is selected to provide from 0.01 weight % to 6.0 weight % hydrogen peroxide to the oral care composition. For example, the amount of cPVP-H₂O₂ complex in the oral care composition may provide from about 0.01 weight % to about 1 weight % hydrogen peroxide, from about 0.01 weight % to about 0.5 weight % hydrogen peroxide, from about 0.05 weight % to about 0.25 weight % hydrogen peroxide, and/or from about 0.1 weight % to about 0.2 weight % hydrogen peroxide in the oral care composition.

In some implementations, the amount of cPVP-H₂O₂ complex in the oral care composition may provide about 0.1 weight % hydrogen peroxide in the oral care composition.

The peroxysulfate whitening agent may include peroxydisultate, peroxydiphosphate, and peroxydicarboate as teeth whitening agents. For example, in one implementation, the peroxysulfate whitening agent includes potassium peroxymonosulfate ("MPS").

An example of a particularly useful peroxysulfate whitening agent is a triple salt mixture comprising potassium hydrogen peroxymonosulfate, potassium hydrogen sulfate, and potassium sulfate. Optionally, such a mixture may further include potassium peroxydilsulfate. An example of such a commercially available mixture is "OXONE", which is the trade name of a mixture sold by DuPont, headquartered in Wilmington, Delaware. Another example is CAROAT, available from United Initiators, headquartered in Pullach, Germany. OXONE consists of 43% potassium hydrogen peroxymonosulfate, 23% potassium hydrogen sulfate, 29% potassium sulfate, 3% potassium peroxidisulfate, and 2% magnesium carbonate. Mixtures of these potassium reagents are usually available as a powder or solid which, when dissolved in water, typically forms a highly acidic solution (e.g., pH 1-4) which is fairly stable on storage. For example, a 1-3% solution of OXONE has a pH of 2.0-2.3. Above pH 6, however, these mixtures are strong oxidizing agents which readily decompose to release reactive oxygen species. Many sources use the terms "potassium hydrogen peroxymonosulfate" or "potassium peroxymonosulfate" to refer to the above triple salt mixture that comprises OXONE (2KHSO₅-KHSO₄-K₂SO₄). As used herein, however, the terms "potassium hydrogen peroxymonosulfate," "potassium peroxymonosulfate," and "MPS" refer to the individual chemical species with the formula KHSO₅.

In one implementation, the amount of peroxysulfate whitening agent is selected to provide from about 0.01 weight % to about 4 weight % MPS to the oral care composition. For example, the amount of peroxysulfate whitening agent in the oral care composition may provide from about 0.01 weight % to about 3 weight % MPS, from about 0.5 weight % to about 3 weight % MPS, from about 1.0 weight % to about 3 weight % MPS, and/or from about 1.5 weight % to about 2.5 weight % MPS in the oral care composition.

In some implementations, the amount of peroxysulfate whitening agent in the oral care composition may provide about 1 weight % MPS in the oral care composition.

In some implementations, the oral care composition may include additional teeth whitening agents selected from: hydrogen peroxide; urea peroxide, sodium percarbonate, sodium perborate; and a combination of two or more thereof.

The oral care composition includes a hydrophobic base. The hydrophobic base may include a hydrophobic polymer, a pressure sensitive adhesive, and a hydrocarbon.

The term "hydrophobic" or "water-insoluble" as applied to polymers and as employed herein refers to an organic polymer which is substantially non-aqueous having a water solubility of less than one gram per 100 grams of water at 25°C, such as a hydrophobic silicone polymer. Any such silicone polymers that are compatible with the whitening agents described herein, and which can produce a tooth whitening composition having the desired viscosity can be used.

The hydrophobic base may include one or more hydrophobic polymers, for example, "siloxane" polymers, which are also generally known in the art as "silicone" polymers. In certain implementations, the hydrophobic polymers are those in which a whitening agent can be dispersed. Many such silicone polymers are commercially available. For example, a preferred silicone-based hydrophobic polymer is a polyorganosiloxane, in particular polydimethylsiloxane (e.g., dimethicone).

The siloxane polymers that can function as part of the hydrophobic base may be in the form of a fluid, such as a polysiloxane fluid. Polysiloxane fluids useful herein include those with a viscosity, at 25° C, of about 1 milliPascal-sec (mPa-s) to about 1000 mPa-s, or about 2 mPa-s to about 500 mPa-s, or about 20 mPa-s to about 400 mPa-s. Polysiloxane fluids for use herein can be linear or cyclic, and can be substituted with a wide variety of substituents. For example, substituents may include methyl, ethyl and phenyl substituents. Suitable polysiloxane fluids include linear polysiloxane polymers, such as, dimethicone and other low viscosity analogues of the polysiloxane materials, having a viscosity, at 25° C., of 200 mPa-s or less and cyclomethicone, and other cyclic siloxanes having for example a viscosity, at 25° C., of 200 mPa-s or less. Other fluids include polysiloxane polyether copolymers and hydroxy terminated polydimethyl-siloxane fluid (e.g., Dow Corning ST-DIMETHICONOL.TM. 40, Dow Corning SGM 36, SGM3). Commercial examples of materials that are suitable for use herein include DC200 series fluids marketed by Dow-Corning Corporation and the AK Fluid series marketed by Wacker-Chemie GmbH, Munchen, Germany. High molecular silicone resins with a polysiloxane blend may also be used including powdered trimethylsiloxysilicate, for example, Dow Corning 593 fluid, Wacker Belsil TMS 803. Another suitable silicone fluid from Dow Corning is Q7-9210.

The hydrophobic base may also include a pressure sensitive adhesive (PSA), such as a silicone pressure sensitive adhesive. Such PSAs can be produced by condensing a silicone resin and an organosiloxane, such as a polydiorganosiloxane. Such PSAs may be an elastomeric, tacky material, adhesion of which to dental enamel surfaces can be varied by altering the ratio of silicone resin to polydiorganosiloxane in the copolymer molecule. Such PSAs may be pressure sensitive hydrophobic polymers specifically designed for pharmaceutical use and are permeable to many drug compounds and find application for the transdermal application of various compounds. The silicone polymers may be the copolymer product of mixing a silanol terminated polydiorganosiloxane, such as polydimethyl siloxane, with a silanol-containing silicone resin whereby the silanol groups of the polydiorganosiloxane undergo a condensation reaction with the silanol groups of the silicone resin so that the polydiorganosiloxane is lightly crosslinked by the silicone resin (that is, the polydiorganosiloxane chains are bonded together through the resin molecules to give chain branching and entanglement and/or a small amount of network character) to form the silicone hydrophobic polymers. A catalyst, for example, an alkaline material, such as ammonia, ammonium hydroxide or ammonium carbonate, can be mixed with the silanol-terminated polydiorganosiloxane and the silicone resin to promote this crosslinking reaction. By copolymerizing the silicone resin with the silanol terminated polydiorganosiloxane, there results a polymer with self-adhering properties and the cohesive properties of a soft elastomer matrix characteristic of pressure sensitive polymers being distinguished from the hard, non-elastomeric properties of other silicone resins. Such PSAs may be available from the Dow-Corning Company under the brand name BIO-PSA. Modifying the silicone resin to polydiorganosiloxane ratio of the PSA will modify the tackiness of the PSA. For example, the BIO PSA silicone adhesive sold by Dow-Corning is available in three silicone resin to silicone polymer ratios namely, 65/35 (low tack), 60/40 (medium tack), 55/45 (high tack) dissolved in either ethyl acetate solvent or dimethicone. A suitable silicone PSA is Silicone Adhesive 8-7016, commercially available from Dow Corning.

In some implementations, the silicone PSA is a copolymer prepared by condensing a silicone resin with a polydiorganosiloxane. In some implementations, the polydiorganosiloxane is polydimethylsiloxane. In some implementations, the silicone resin is a silanol-containing silicone resin.

The hydrophobic base may further include a natural or synthetic hydrocarbon, such as mineral oil, petrolatum, or liquid paraffin, e.g., white petrolatum. In some implementations, the oral care composition includes from about 10 weight % to about 30 weight % of a natural or synthetic hydrocarbon, e.g., from about 15 weight % to about 25 weight %.

The oral care composition includes from 20 weight % to 80 weight % hydrophobic base based on the total weight of the oral care composition. For example, the oral care composition may include from about 40 weight % to 80 weight % hydrophobic base, from about 60 weight % to 80 weight % hydrophobic base, from about 70 weight % to 80 weight % hydrophobic base, and/or from about 70 weight % to about 75 weight % hydrophobic base. In other implementations, the hydrophobic base includes a PSA, and the oral care composition may include from 20 weight % to 80 weight % PSA, or about 30 % PSA, based on the total weight of the oral care composition.

In some implementations, the hydrophobic base includes one or more hydrophobic polymers, and the oral care composition includes from about 30 weight % to about 70 weight % hydrophobic polymer, 40 weight % to about 60 weight % hydrophobic polymer, 45 weight % to about 55 weight % hydrophobic polymer, 45 weight % to about 50 weight % hydrophobic polymer, or about 55 weight % hydrophobic polymer, based on a total weight of the oral care composition.

The hydrophobic base may consist of one or more silicone polymers combined with a natural or synthetic hydrocarbon. In some implementations, the hydrophobic base does not include polyethylene thickener (including polyethylene/mineral oil blends, also known as Plastigels).

The present inventors have discovered that particular amounts of a dental surface adhesion enhancing agent not only provides greater retention of the oral care composition to the tooth surface, but also enhances the stability of the peroxide and/or peroxysulfate whitening agents and helps to maximize delivery of an effective concentration of the peroxide and/or peroxysulfate whitening agents at the target site. Dental surface adhesion agents are polar organic polymers which, due to their polarity, tend to adhere electrostatically to the surface of the tooth. Dental surface adhesion agents may be any polar organic polymers, including anionic polymers, cationic polymers, and neutral polar polymers. Suitable anionic polymers include polyacrylate polymers and copolymers of methyl vinyl ether with maleic acid or anhydride. Suitable neutral polar polymers include polyvinylpyrrolidone (PVP), including either linear PVP or cross-linked PVP (cPVP), or a mixture thereof. In some implementations, the total amount of dental surface adhesion agent in the oral care composition ranges from about 10 weight % to about 40 weight %, based on the total weight of the oral care composition. Such concentration of dental surface adhesion agent includes the cPVP present in the cPVP-hydrogen peroxide complex as described herein. In some implementations, the only dental surface adhesion agent present is PVP, e.g., linear PVP, cPVP or a mixture thereof (including the cPVP present in the cPVP-peroxide complex).

In some implementations, the total amount of dental surface adhesion agent, e.g., PVP (including linear PVP, cPVP or mixtures thereof, including the cPVP in cPVP-H₂O₂) is from about 10 weight % to about 30 weight %, or from about 15 weight % to about 25 weight %, or from about 20 weight % to about 25 weight %, or about 20 weight %, or about 22 weight % or about 24 weight %, by total weight of the oral care composition. In some implementations, the only source of dental surface adhesion agent is the cPVP present in the cPVP-hydrogen peroxide complex. In other implementations, substantially all of the dental surface adhesion agent (e.g., from about 95 weight % to about 99 weight % thereof) is provided by added dental surface adhesion agent (e.g., added cPVP, linear PVP, or a mixture thereof).

Adhesiveness may be measured using standard adhesion tests known in the art, for example, the adhesive test disclosed in U.S. Pat. No. 6,613,812 to Bui. In certain implementations, the adhesiveness between a tooth and a film formed from an oral care composition according to the present disclosure may be from about at least 500 pounds per square inch (PSI), at least 1,000 PSI, at least 2,000 PSI, or greater.

According to some implementations, the inclusion of sodium tripolyphosphate (STPP) in the oral care composition, in an amount from 0.05 weight % to 3 weight %, based on the total weight of the oral care composition, results in improved resistance to microbial growth and contamination (microrobustness) and improved whitening effectiveness. Optionally, the concentration of sodium tripolyphosphate is from about 0.1 weight % to about 2 weight %, or from about 0.1 weight % to about 0.5 weight %, or from about 1.5 weight % to about 2 weight %, or about 0.1 weight % or about 2 weight %. While common oral care products typically contain an antimicrobial or preservative agent to inhibit the microbial growth that can cause spoilage of toothpastes and other dentifrice products, examples of such agents include quaternary ammonium salts, such as cetylpyridinium chloride (CPC), and zinc ion sources, such as zinc oxide. However, the present inventors have unexpectedly found that the inclusion of appropriate concentrations of STPP, as provided herein, helps contribute to microrobustness in these oral care compositions sufficiently that no added antimicrobial agent or preservative is needed. Without being bound by theory, it is believed that STPP chelates metal ions that are important in microbial metabolism, thus inhibiting microbial growth. Thus, in some implementations, the oral care composition does not include antimicrobial agents or preservative agents (e.g., CPC and zinc oxide). In one preferred implementation, the oral care composition does not include CPC. STPP also provides important benefits in prevent stain formation on the teeth. Moreover, CPC has been shown to contribute to stain formation in some cases, due its cationic character, and the inclusion of STPP and the absence of CPC further contributes to the prevention of teeth staining.

As described above, the oral care composition may include additional ingredients common to oral care compositions. For example, the oral care composition may include a tartar control or anti-calculus agent, in addition to the aforementioned sodium tripolyphosphate. Such agent are also useful as stain-prevention agents in the present oral care compositions. Such agents include salts of any of these agents, for example their alkali metal and ammonium salts: phosphates and polyphosphates (for example pyrophosphates), polyaminopropanesulfonic acid (AMPS), polyolefin sulfonates, polyolefin phosphates, diphosphonates such as azacycloalkane-2,2-diphosphonates (e.g., azacycloheptane-2,2-diphosphonic acid), N-methyl azacyclopentane-2,3-diphosphonic acid, ethane-1-hydroxy-1,1-diphosphonic acid (EHDP) and ethane-1-amino-1,1-diphosphonate, phosphonoalkane carboxylic acids and. Useful inorganic phosphate and polyphosphate salts include monobasic, dibasic and tribasic sodium phosphates, sodium tripolyphosphate, tetrapolyphosphate, mono-, di-, tri- and tetrasodium pyrophosphates, sodium trimetaphosphate, sodium hexametaphosphate (SHMP) and mixtures thereof. In a particular implementation, SHMP is used. The amount of such agent optionally present is from about 0.1 weight % to about 10 weight %, from about 2 weight % to about 9 weight %, and from about 5 weight % to about 8 weight %, or about 7 weight %, based on the total weight of the oral care composition.

The oral care compositions may also include a flavoring agent. Suitable flavoring agents include, but are not limited to, essential oils as well as various flavoring aldehydes, esters, alcohols, and similar materials. Examples of the essential oils include oils of spearmint, peppermint, wintergreen, sassafras, clove, sage, eucalyptus, marjoram, cinnamon, lemon, lime, grapefruit, and orange. Also useful are such chemicals as menthol, carvone, and anethole. Of these, the most commonly employed are the oils of peppermint, spearmint and wintergreen. The flavoring agent is incorporated in the oral care compositions of the present invention at a concentration of about 0.01 weight % to about 2 weight %, and preferably from about 0.1 weight % to about 0.5 weight %.

Further implementations provide a method for whitening a tooth including applying an oral care composition according to the present disclosure to a tooth of a mammal. In some implementations, the oral care composition is applied using a pen. In some implementations, the oral care composition is maintained on the surface of the tooth for a plurality of minutes. Further implementations provide use of any of the oral care compositions described herein in any one of the methods for whitening a tooth, as described herein.

In some implementations, the oral care composition is maintained on the surface of a tooth for from about 1 minute to about 8 hours. In some implementations, the oral care composition is maintained on the surface of a tooth for from about 5 minutes to about 4 hours. In some implementations, the oral care composition is maintained on the surface of a tooth for from about 10 minutes to about 120 minutes. In some implementations, the oral care composition is maintained on the surface of a tooth for from about 15 minutes to about 60 minutes. In some implementations, the oral care composition is maintained on the surface of a tooth for from about 20 minutes to about 45 minutes. In some implementations, the oral care composition is maintained on the surface of a tooth for about 30 minutes.

In some implementations, the oral care composition is in the form of a gel.

In some implementations, the oral care compositions do not phase separate to an unacceptable level after 1 month.

In some implementations, the oral care composition has a Hershel-Bulkley rate index of less than 0.7. In some implementations, the oral care composition has a Hershel-Bulkley rate index of less than 0.68. In some implementations, the oral care composition has a Hershel-Bulkley rate index of less than 0.65.

In some implementations, the oral care composition has a G'/G" ratio of greater than or equal to 1 in the linear viscoelastic region. In some implementations, the oral care composition has a G'/G" ratio of greater than or equal to 1.5 in the linear viscoelastic region. In some implementations, the oral care composition has a G'/G" ratio of greater than or equal to 2 in the linear viscoelastic region.

In some implementations, the oral care composition has a critical strain greater than or equal to 0.02.

In some implementations, the oral care composition has a Hershel-Bulkley rate index of less than 0.7; a G'/G" ratio of greater than or equal to 1 in the linear viscoelastic region; and a critical strain greater than or equal to 0.02.

In some implementations, the oral care compositions of the present disclosure can be prepared by adding and mixing the ingredients of the oral care composition in a suitable vessel, such as a stainless steel tank, provided with a mixer. In the preparation of the oral care compositions described herein, the ingredients are advantageously added to the mixer in the following order: hydrophobic component, dental surface adhesion enhancing agent, peroxide component, peroxysulfate component, and any desired flavoring or sweetener. The ingredients are then mixed to form a homogeneous dispersion/solution.

In some implementations, the oral care compositions are applied to the tooth of a subject, by manual application, such as by painting the teeth with a soft applicator brush in the same manner as application of nail polish to a finger nail and without the intervention of a dentist or technological operations. Application by the user, leaves a coating of the thick liquid suspension on the teeth. Contact with saliva causes the slow release of H₂O₂ from the matrix of the peroxide component and permits efficient delivery of the peroxide source to the target site, e.g. the tooth.

Typically, the oral care compositions are applied directly to the teeth, e.g., by painting the teeth for a time sufficient to effect whitening. The oral care compositions of the present disclosure can be used in a regimen for whitening teeth and can be used in combination with a whitening toothpaste and a whitening mouthwash to further enhance the whitening results.

Some implementations provide a method wherein the applicator is a pen and the pen is stored within an oral care implement. In some implementations, the pen is removed from the oral care implement prior to application of the oral care composition to the tooth. In some implementations, the oral care composition is applied to the tooth after brushing. In some implementations, the oral care composition is applied to the tooth after brushing with the oral care implement.

As used herein, "whitening" refers to a change in visual appearance of a tooth, preferably such that the tooth has a brighter shade. Increase in whiteness of a dental surface can be observed visually, for example with the aid of color comparison charts or gauges, or measured by colorimetry, using any suitable instrument such as a Minolta Chromameter, e.g., model CR-400 (Minolta Corp., Ramsey, N.J.). The instrument can be programmed, for example, to measure Hunter Lab values or L*a*b* values according to the standard established by the International Committee of Illumination (CIE). The L*a*b* system provides a numerical representation of three-dimensional color space where L* represents a lightness axis, a* represents a red-green axis and b* represents a yellow-blue axis. The L* and b* axes are typically of greatest applicability to measurement of tooth whiteness. Increase in whiteness can be computed from differences in L*, a* and b* values before and after treatment, or between untreated and treated surfaces.

As used herein, "tooth" or "teeth" refers to natural mammalian teeth, dentures, dental plates, fillings, caps, crowns, bridges, dental implants, and the like, and any other hard surfaced dental prosthesis either permanently or temporarily fixed within the oral cavity.

### EXAMPLES

Aspects of the present disclosure may be further understood by referring to the following examples. The examples are illustrative.

Table 1 (below) provides the formulations for an exemplary oral care composition according to the present disclosure and two comparative compositions. All three compositions include about 0.1 weight % hydrogen peroxide as a whitening agent and have similar ingredients/amounts. However, Comparative Composition #1 includes CPC as an antibacterial or preservative agent, while Comparative Composition #2 includes STPP as the antibacterial or preservative agent. Oral Care Composition #1 includes a combination of STPP and MPS.

**Table 1**

| | **Comparative Composition #1** | **Comparative Composition #2** | **Oral Care Composition #1** |
|---|---|---|---|
| **Ingredient** | **Wt. %** | **Wt. %** | **Wt. %** |
| Silicon PSA | 30.00 | 30.00 | 30.00 |
| White Petrolatum | 25.00 | 25.00 | 25.00 |
| Polyvinyl pyrrolidone | 24.35 | 22.35 | 22.15 |
| Silicon Polymer | 18.10 | 18.00 | 16.20 |
| Fumed Silica | 1.00 | 1.00 | 1.00 |
| Whitening Flavor | 0.60 | 0.80 | 0.80 |
| Peroxydone XL (18 % Hydrogen Peroxide) | 0.55 | 0.55 | 0.55 |
| Sodium Saccharin | 0.30 | 0.30 | 0.30 |
| CPC | 0.10 | - | - |
| STPP | - | 2.00 | 2.00 |
| OXONE (50% MPS) | - | - | 2.00 |
| Total Components | 100.00 | 100.00 | 100.00 |

Oral Care Composition #1 and Comparative Composition #1 were then tested to measure their whitening efficacy via an in-vitro whitening assay. Briefly, mounted bovine teeth were submerged in 50 mM phosphate buffer, then removed and coated for 15 minutes with both compositions. The compositions were removed from the teeth using laboratory wipes and then were rinsed with deionized water. Each such treatment cycle was repeated 42 times. Spectrophotometer measurements were taken before the first treatment (baseline) and after the seventh, fourteenth, twenty-first, twenty-eighth, thirty-fifth and forty-two treatments. L*, a* and b* values were measured and a whiteness score, W* was calculated. W* is a measure of overall color change relative to pure white. The applicable formula is W* = ((a*)² + (b*)² + (L*-100)²)^{½}. ΔW is the difference in whiteness score between the test measure and the baseline score. The results of the whitening efficacy tests are illustrated in Table 2.

**Table 2**

| ΔW | No. of Treatments | | | | | | |
|---|---|---|---|---|---|---|---|
| | 0 | 7 | 14 | 21 | 28 | 35 | 42 |
| Comparative Composition #1 | 0.0 | -0.2 | -0.9 | -0.8 | -1.0 | -1.7 | -1.4 |
| Oral Care Composition #1 | 0.0 | -0.7 | -1.1 | -1.4 | -1.8 | -3.0 | -3.2 |

As illustrated in Table 2, teeth brushed using Oral Care Composition #1 had a lower ΔW value when compared to teeth brushed using the Comparative Composition #1. Table 2 demonstrates the superior whitening efficacy of oral care compositions according to implementations of the present disclosure.

The ability of Oral Care Composition #1, Comparative Composition #1, and Comparative Composition #2 to withstand a microbial challenge was then measured using a short-term kill challenge test which uses a pool of various ATCC and environmental microorganism strains. Samples were challenged three times each by the bacterial pool. The inoculated samples were plated to measure the log reduction of bacteria over 24 hours. Using that data, the area under the curve (AUC) was calculated and then normalized versus a standard to generate a robustness score; the higher the score, the greater the microbial robustness of the composition. The results are summarized in Table 3.

**Table 3**

| | **Comparative Composition #1** | **Comparative Composition #2** | **Oral Care Composition #1** |
|---|---|---|---|
| **Micro Robustness Index (MRI)** | 0.70 | 0.77 | 1.00 |

As illustrated in Table 3, Oral Care Composition #1 had a much higher score when compared to Comparative Composition #1 and Comparative Composition #2. Table 3 demonstrates the superior micro robustness of Oral Care Composition #1, and in particular, the synergistic effects of oral care compositions according to the present disclosure against composition using only STPP or CPC as microbial agents.

The present disclosure has been described with reference to exemplary implementations.

## Claims

1. An oral care composition, comprising:
a peroxide source comprising a cross-linked polyvinylpyrrolidone-hydrogen peroxide complex (cPVP-H₂O₂ complex) in an amount configured to provide from 0.01 weight % to 6.0 weight % hydrogen peroxide based on a total weight of the oral care composition;
a peroxysulfate whitening agent;
from 20 weight % to 80 weight % of a hydrophobic base based on the total weight of the oral care composition; and
from 0.05 weight % to 3 weight % sodium tripolyphosphate (STPP).

2. The oral care composition of claim 1, wherein the peroxysulfate whitening agent comprises potassium peroxymonosulfate (MPS), and wherein an amount of the peroxysulfate whitening agent is configured to provide from 0.01 weight % to 4 weight % MPS based on the total weight of the oral care composition.

3. The oral care composition of claim 1, wherein the hydrophobic base comprises one or more of a hydrophobic polymer, a pressure sensitive adhesive, and a hydrocarbon.

4. The oral care composition of claim 3, wherein the hydrophobic polymer comprises polydimethylsiloxane.

5. The oral care composition of claim 3, wherein the pressure sensitive adhesive is a silicone copolymer prepared by condensing a silicone resin with a polydiorganosiloxane and wherein the hydrocarbon is petrolatum.

6. The oral care composition of claim 3, wherein the hydrophobic base comprises:
from 30 weight % to 70 weight % hydrophobic polymer based on the total weight of the oral care composition;
from 20 weight % to 40 weight % pressure sensitive adhesive based on the total weight of the oral care composition;
from 10 weight % to 30 weight % hydrocarbon based on the total weight of the oral care composition; and
from 10 weight % to 30 weight % of a dental surface adhesion agent.

7. The oral care composition of claim 6, wherein the dental surface adhesion agent comprises a polyvinylpyrrolidone (PVP) polymer, including either linear PVP or cross-linked PVP (cPVP), or a mixture thereof.

8. The oral care composition of claim 7, wherein the dental surface adhesion agent is one or more of anionic polymers, cationic polymers, and neutral polar polymers, such as polyacrylate polymers, copolymers of methyl vinyl ether with maleic acid or anhydride, and linear PVP or cross-linked PVP, and any mixtures thereof.

9. The oral care composition of claim 1, wherein the oral care composition has less than 5 weight % water based on the total weight of the oral care composition.

10. The oral care composition of claim 1, wherein the oral care composition does not comprise a surfactant, a polyethylene thickener, or a source of fluoride ions.

11. The oral care composition of claim 1, wherein the oral care composition does not comprise cetylpyridinium chloride, and wherein the oral care composition does not comprise any antimicrobial or preservative agents other than STPP.

12. The oral care composition of claim 1, wherein the oral care composition is embodied as a gel configured to be maintained on a surface of a tooth for from 10 minutes to 120 minutes.

13. A method of whitening a tooth comprising applying the oral care composition of claim 1 to a tooth of a mammal.

14. The method of claim 13, wherein the oral care composition is sufficiently viscous to form an adherent, continuous layer on a dental surface and deliver an effective amount the peroxide source and the peroxysulfate whitening agent to a tooth surface.

## Patentansprüche

1. Mundpflegezusammensetzung, umfassend:
eine Peroxidquelle, die einen vernetzten Polyvinylpyrrolidon-Wasserstoffperoxid Komplex (cPVP-H₂O₂-Komplex) in einer Menge umfasst, die so ausgelegt ist, dass 0,01 Gewichts-% bis 6,0 Gewichts-% Wasserstoffperoxid, bezogen auf das Gesamtgewicht der Mundpflegezusammensetzung, bereitgestellt werden;
ein Peroxysulfat-Aufhellungsmittel;
von 20 Gewichts-% bis 80 Gewichts-% einer hydrophoben Base, bezogen auf das Gesamtgewicht der Mundpflegezusammensetzung; und
von 0,05 Gewichts-% bis 3 Gewichts-% Natriumtripolyphosphat (STPP).

2. Mundpflegezusammensetzung nach Anspruch 1, wobei das Peroxysulfat-Aufhellungsmittel Kaliumperoxymonosulfat (MPS) umfasst, und wobei eine Menge des Peroxysulfat-Aufhellungsmittels so ausgelegt ist, dass 0,01 Gewichts-% bis 4 Gewichts-% MPS, bezogen auf das Gesamtgewicht der Mundpflegezusammensetzung, bereitgestellt werden.

3. Mundpflegezusammensetzung nach Anspruch 1, wobei die hydrophobe Base eines oder mehrere von einem hydrophoben Polymer, einem Haftkleber und einem Kohlenwasserstoff umfasst.

4. Mundpflegezusammensetzung nach Anspruch 3, wobei das hydrophobe Polymer Polydimethylsiloxan umfasst.

5. Mundpflegezusammensetzung nach Anspruch 3, wobei der Haftklebstoff ein Silikon-Copolymer ist das durch Kondensation eines Silikonharzes mit einem Polydiorganosiloxan hergestellt wird, und wobei der Kohlenwasserstoff Petrolatum ist.

6. Mundpflegezusammensetzung nach Anspruch 3, wobei die hydrophobe Base umfasst:
30 Gewichts-% bis 70 Gewichts-% hydrophobes Polymer, bezogen auf das Gesamtgewicht der Mundpflegezusammensetzung;
20 Gewichts-% bis 40 Gewichts-% Haftkleber, bezogen auf das Gesamtgewicht der Mundpflegezusammensetzung;
10 Gewichts-% bis 30 Gewichts-% Kohlenwasserstoff, bezogen auf das Gesamtgewicht der Mundpflegezusammensetzung; und
10 Gewichts-% bis 30 Gewichts-% eines Adhäsionsmittels für dentale Oberflächen.

7. Mundpflegezusammensetzung nach Anspruch 6, wobei das Adhäsionsmittel für dentale Oberflächen ein Polyvinylpyrrolidon (PVP)-Polymer umfasst, das entweder lineares PVP oder vernetztes PVP (cPVP) oder eine Mischung davon enthält.

8. Mundpflegezusammensetzung nach Anspruch 7, wobei das Adhäsionsmittel für dentale Oberflächen eines oder mehrere von anionischen Polymeren, kationischen Polymeren und neutralen polaren Polymeren, wie Polyacrylatpolymere, Copolymere von Methylvinylether mit Maleinsäure oder -anhydrid und lineares PVP oder vernetztes PVP und beliebige Mischungen davon ist.

9. Mundpflegezusammensetzung nach Anspruch 1, wobei die Mundpflegezusammensetzung weniger als 5 Gewichts-% Wasser, bezogen auf das Gesamtgewicht der Mundpflegezusammensetzung, aufweist.

10. Mundpflegezusammensetzung nach Anspruch 1, wobei die Mundpflegezusammensetzung kein oberflächenaktives Mittel, kein Polyethylen-Verdickungsmittel und keine Fluoridionenquelle umfasst.

11. Mundpflegezusammensetzung nach Anspruch 1, wobei die Mundpflegezusammensetzung kein Cetylpyridiniumchlorid umfasst, und wobei die Mundpflegezusammensetzung keine anderen antimikrobiellen oder konservierenden Mittel als STPP umfasst.

12. Mundpflegezusammensetzung nach Anspruch 1, wobei die Mundpflegezusammensetzung als Gel ausgebildet ist, das so ausgelegt ist, dass es 10 Minuten bis 120 Minuten auf einer Zahnoberfläche verbleibt.

13. Verfahren zum Aufhellen eines Zahns, umfassend das Auftragen der Mundpflegezusammensetzung nach Anspruch 1 auf einen Zahn von einem Säugetier.

14. Verfahren nach Anspruch 13, wobei die Mundpflegezusammensetzung ausreichend viskos ist, um eine haftende, kontinuierliche Schicht auf einer dentalen Oberfläche zu bilden und eine wirksame Menge der Peroxidquelle und des Peroxysulfat-Aufhellungsmittels an eine Zahnoberfläche abzugeben.

## Revendications

1. Composition de soins bucco-dentaires comprenant :
une source de peroxyde comprenant un complexe polyvinylpyrrolidone réticulée-peroxyde d'hydrogène (complexe cPVP-H₂O₂) en une quantité conçue pour fournir de 0,01 % en poids à 6,0 % en poids de peroxyde d'hydrogène par rapport au poids total de la composition de soins bucco-dentaires ;
un agent de blanchiment à base de peroxysulfate ;
de 20 % en poids à 80 % en poids d'une base hydrophobe par rapport au poids total de la composition de soins bucco-dentaires ; et
de 0,05 % en poids à 3 % en poids de tripolyphosphate de sodium (STPP).

2. Composition de soins bucco-dentaires selon la revendication 1, dans laquelle l'agent de blanchiment à base de peroxysulfate comprend du peroxymonosulfate de potassium (MPS), et dans laquelle une quantité d'agent de blanchiment à base de peroxysulfate est conçue pour fournir de 0,01 % en poids à 4 % en poids de MPS par rapport au poids total de la composition de soins bucco-dentaires.

3. Composition de soins bucco-dentaires selon la revendication 1, dans laquelle la base hydrophobe comprend un ou plusieurs éléments parmi un polymère hydrophobe, un adhésif sensible à la pression et un hydrocarbure.

4. Composition de soins bucco-dentaires selon la revendication 3, dans laquelle le polymère hydrophobe comprend du polydiméthylsiloxane.

5. Composition de soins bucco-dentaires selon la revendication 3, dans laquelle l'adhésif sensible à la pression est un copolymère de silicone préparé par condensation d'une résine de silicone avec un polydiorganosiloxane et dans laquelle l'hydrocarbure est de la vaseline.

6. Composition de soins bucco-dentaires selon la revendication 3, dans laquelle la base hydrophobe comprend :
de 30 % en poids à 70 % en poids de polymère hydrophobe par rapport au poids total de la composition de soins bucco-dentaires ;
de 20 % en poids à 40 % en poids d'adhésif sensible à la pression par rapport au poids total de la composition de soins bucco-dentaires ;
de 10 % en poids à 30 % en poids d'hydrocarbure par rapport au poids total de la composition de soins bucco-dentaires ; et
de 10 % en poids à 30 % en poids d'un agent d'adhérence à la surface dentaire.

7. Composition de soins bucco-dentaires selon la revendication 6, dans laquelle l'agent d'adhérence à la surface dentaire comprend un polymère de polyvinylpyrrolidone (PVP), notamment du PVP linéaire ou du PVP réticulé (cPVP), ou un mélange de ceux-ci.

8. Composition de soins bucco-dentaires selon la revendication 7, dans laquelle l'agent d'adhérence à la surface dentaire est un ou plusieurs polymères parmi les polymères anioniques, les polymères cationiques et les polymères polaires neutres, tels que les polymères de polyacrylate, les copolymères de méthylvinyléther avec l'acide ou l'anhydride maléique, le PVP linéaire ou le PVP réticulé, et tous les mélanges de ceux-ci.

9. Composition de soins bucco-dentaires selon la revendication 1, dans laquelle la composition de soins bucco-dentaires présente moins de 5 % en poids d'eau par rapport au poids total de la composition de soins bucco-dentaires.

10. Composition de soins bucco-dentaires selon la revendication 1, dans laquelle la composition de soins bucco-dentaires ne comprend ni tensioactif, ni épaississant polyéthylène, ni source d'ions fluorure.

11. Composition de soins bucco-dentaires selon la revendication 1, dans laquelle la composition de soins bucco-dentaires ne comprend pas de chlorure de cétylpyridi-nium, et dans laquelle la composition de soins bucco-dentaires ne comprend ni agents antimicrobiens, ni agents conservateurs autres que le STPP.

12. Composition de soins bucco-dentaires selon la revendication 1, dans laquelle la composition de soins bucco-dentaires se présente sous la forme d'un gel conçu pour être maintenu sur la surface d'une dent pendant 10 minutes à 120 minutes.

13. Procédé de blanchiment d'une dent comprenant l'application de la composition de soins bucco-dentaires selon la revendication 1 sur une dent d'un mammifère.

14. Procédé selon la revendication 13, dans lequel la composition de soins bucco-dentaires est suffisamment visqueuse pour former une couche continue et adhérente sur une surface dentaire et distriburer une quantité efficace de la source de peroxyde et de l'agent de blanchiment à base de peroxysulfate à la surface d'une dent.
